# NEW EUROPEAN PATENT SPECIFICATION

(11) **EP 0 305 364 B2**
(45) Date of publication and mention of the opposition decision: **07.01.1998**
(45) Mention of the grant of the patent: 24.02.1993
(21) Application number: 87901905.7
(22) Date of filing: 25.02.1987
(51) Int. Cl.: A61M 1/00, A61M 37/00

(54) **PURGING SYSTEM FOR A BLOOD TUBING NETWORK**
REINIGUNGSSYSTEM FÜR EIN BLUTLEITUNGSNETZ
SYSTEME DE PURGE POUR UN RESEAU DE TUYAUX TRANSPORTANT DU SANG

(43) Date of publication of application: 08.03.1989
(73) Proprietor: BAXTER INTERNATIONAL INC. (a Delaware corporation), Deerfield Illinois 60015 (US)
(72) Inventor: WILLIAMSON,warren, EL TORO CA 92630 2526 (US); PRINCE, Paul, R., Fountain Valley, CA 92708 (US)
(74) Representative: MacGregor, Gordon
(86) International application number: US8700353
(87) International publication number: WO8806460

(56) References cited:
- EP-A- 0 161 686
- EP-A- 0 203 513
- DE-A- 3 442 744
- FR-A- 2 566 273
- US-A- 2 309 302
- US-A- 2 452 643
- US-A- 2 817 372
- US-A- 2 833 281
- US-A- 3 017 885
- US-A- 3 833 013
- US-A- 4 177 808
- US-A- 4 428 743
- US-A- 4 437 874
- US-A- 4 445 884
- US-A- 4 447 237

## Description

### FIELD OF INVENTION

The present invention generally relates to method and apparatus for purging air from a disposable tubing set (e.g., of the type used in a blood constituent processing system which both withdraws and returns blood constituents from/to a patient). It also generally relates to method and apparatus for maintaining the needle(s) of such a system in a sterile or antiseptic environment until actual usage.

### BACKGROUND AND SUMMARY OF THE INVENTION

Conventional blood constituent processing systems typically utilize a disposable flexible tubing network (including connected related devices) for withdrawing blood from a donor, processing the blood (for example, to separate a desired blood constituent) and then returning processed blood constituents to the patient or donor. Blood constituent processing systems may, for example, be of the type which pass blood through a constituent separating device so as to obtain concentrated blood plasma and/or platelet constituents while returning residual blood constituents to the donor or making them available for other uses. While some systems use a single needle for withdrawal and return (e.g., on a time multiplexed basis), others use a pair of needles -- one for withdrawal and one for return.

In such blood processing systems, it is, of course, necessary that air be purged from the tubing network prior to connection to a human patient or donor. It would also be advantageous for air to be purged automatically (as by the execution of a suitable computer program which effects control over fluid pumps, valves, etc., operatively acting on the network after its installation in a host machine) to minimize the phlebotomist's tasks.

Until the present invention, one conventional purge procedure has been simply to pass a purge fluid (i.e., a saline solution) through the tubing network until the fluid is discharged from the distal end of the collection/return needles with the discharged fluid being collected in a suitable waste receptacle. The saline solution is discharged from the needles until the phlebotomist is satisfied that all air has been purged in the upstream tubing network. This is not only a messy and wasteful procedure, it also necessarily exposes the needle to a nonsterile atmosphere throughout the procedure.

It is also known to provide a single tubular needle with a frangible sheath including a porous biological filter of the type which is permeable to gas (e.g., air) yet hydrophobic and thus impermeable to liquid (e.g., saline solution and/or blood). While enclosing a single needle with a biological filter/sheath may be adequate for relatively simple tubing networks -- that is, tubing networks having few, if any, branches and/or flow paths -- such a conventional system is inadequate for a relatively complex tubing network having numerous branches and/or flow paths which must be purged of air prior to use. The possibility exists in complex tubing networks for multiple "pockets" of air to be interspersed with the saline purge liquid. Thus, once an advance column of purge liquid reaches the biological filter sheath, air entrained upstream will not be permitted to reach the filter (and thus be purged from the tubing network) since the advance liquid column will, in effect, "lock" further flow towards the filter due to its impermeability to liquid.

To alleviate this problem and thus ensure complete air purging, it has been prior practice to circulate saline in a portion of the tube set and then to simply remove the biological filter/sheath so as to permit remaining upstream columns of saline liquid (and thus the remaining pockets of entrained air) to be discharged from the needle. Accordingly, for all intents and purposes, there is typically only one conventional method of removing remaining pockets of entrained air from a complex tubing network -- that is, by simply allowing a sterile liquid (e.g., saline) to freely flow through the tubing network and be discharged from the needle for a time sufficient to ensure complete air purging. FR-A-2566273 discloses a method and apparatus for passing a detergent or washing solution held in a multi-chamber receptacle into the flow paths and filters of dialysis equipment such that any air in the flow path would inherently collect in the air space of one of the chambers.

Prolonged exposure of the collection/return needles to a nonsterile environment is, for obvious reasons, undesirable. Yet the relevant prior practices described above tend to exacerbate potential contamination of the needle since exposure to a nonsterile environment occurs during much of the purging cycle -- and may continue even thereafter if the patient/donor is not accessible for immediate attachment after the purge cycle is complete. While it is perhaps physically impossible to maintain the needles in a completely sterile environment prior to their percutaneous entry into a patient or donor, it is highly desirable to minimize the time during which the needles are exposed to a non-sterile environment.

The present invention simultaneously accomplished such goals in a dual needle type of system by commonly enclosing the collection and return needles in a sterile housing that can also be used to facilitate the purging cycle. Thus, saline liquid during the purge cycle is permitted to flow serially through the needles and the common enclosure the air being then collected (or purged) in the enclosure itself and/or in a separate air trap location of the tubing network.

The tubing network including the collection/return needles, their common enclosure and blood constituent processing devices (e.g., blood filters, platelet separators, etc.) can thus be manufactured as a unitary disposable unit which can be connected to an automatic blood processing unit prior to use. Moreover, since the tubing network will remain "closed" during the purge sequence, the tubing network is particularly adapted for automated purging of air. Once all air has been purged from the tubing network, the phlebotomist may wait until the patient/donor is at hand and prepared before separating the collection and/or return needle(s) from their enclosure and intravenously connecting them. The enclosure thus not only facilitates purging, it also maintains the needle(s) in a sterile environment to minimize the time interval in which the needles are exposed to a nonsterile environment.

The advantages and objects briefly mentioned above, as well as others, will be more clearly understood after careful consideration is given to the detailed descriptions of exemplary embodiments of this invention which follow.

### BRIEF DESCRIPTION OF THE ACCOMPANYING DRAWINGS

Reference will hereinafter be made to the accompanying drawings wherein like reference numerals throughout the various FIGURES denote like structural elements and wherein:
FIGURE 1 is a schematic representation of a blood constituent processing system employing the present invention;
FIGURE 2 is an elevational view, partly in section, of an exemplary embodiment of a dual needle housing/priming device in accordance with this invention;
FIGURE 3 is a detailed elevational view taken along line 3-3 in FIGURE 2;
FIGURE 4 is an elevational view, partly in section, showing another embodiment of the invention; and
FIGURE 5 is a cross-sectional view of a further embodiment of the invention.

### DETAILED DESCRIPTION OF THE EXEMPLARY EMBODIMENTS

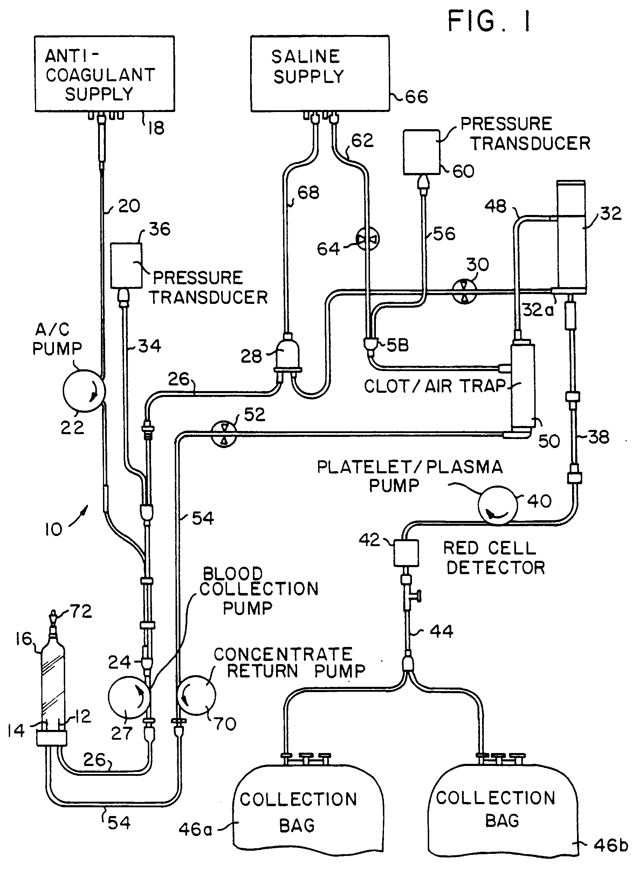

A blood constituent processing system 10 is shown in FIGURE 1 as employing a tubing network having collection and return needles 12, 14, respectively, commonly housed within an enclosure 16. In the exemplary system of FIGURE 1, two needles are employed -- that is, one needle 12 is employed as a collection needle (used to collect whole blood from a patient or donor) and a second needle 14 is employed as a return needle (used to return residual/treated blood constituents to the donor through e.g., a vein located in a different part of the body from the location of collection needle 12). Additional needles may be employed and also commonly housed within enclosure 16 for other functions (e.g., monitoring the patient's or donor's pulse rate, blood pressure, etc., by means not shown in FIGURE 1).

The entire fluid flow path (including all interconnecting tubing, the platelet separator/plasma filter and the like) is preferably defined by a disposable plastic tubing network or set which also includes the collection and return needles 12, 14 commonly housed within enclosure 16. The disposable tubing network is first manually inserted into an automatic blood processing apparatus 10 so as to be mechanically coupled to various computer-controlled peristaltic pumps, pressure sensors, electromagnetically operated clamps, and the like. The phlebotomist may, after priming, then withdraw needles 12, 14 from enclosure 16 and intravenously connect them to the patient/donor so that blood processing may commence.

During blood processing, an anticoagulant is typically fed from supply 18 through line 20 by controlled pump 22 into the drawn blood supply at a location 24 near the collection needle 12. Drawn blood is pumped through the collection needle 12 in line 26 by means of controlled peristaltic blood pump 27, through an air bubble trap 28, an open blood clamp 30 and to the input side 32a of platelet separator/plasma filter device 32. A pressure transducer branch tubing 34 communicates with pressure transducer 36. The filtrate from separator/filter 32 may be pumped through line 38 by controlled peristaltic platelet/plasma pump 40 (or merely permitted to flow as the difference between the controlled flow rates of pumps 27 and 70 as should be apparent) and on through a photosensitive red cell detector 42 and tubing 44 to filtrate collection bags 46a and 46b.

The residual (e.g., concentrated) blood constituents from separator/filter 32 is pumped by concentrate return pump 70 to pass out through line 48, a blood clot/air trap 50, an open return clamp 52 and back to the return needle 14 via tubing 54. The pressure of return line 54 may be monitored via branch line 56 connected at junction 55 communicating with pressure transducer 60. A saline branch line 62 connects near junction 58 passing through electromagnetically controlled clamp 64 and back to the saline supply 66 (which is also connectable via tubing 68 to the filter trap 28 and, therefore, to the collection side of the fluid circuit).

Prior to actual blood processing and with the tubing set manually interconnected to the automatic processing apparatus 10, the phlebotomist may select an automatic priming sequence with common enclosure 16 still in place and enclosing collection and return needles 12, 14. During such initial priming procedures (which may include successive programmed pumping of saline solution bidirectionally throughout various parts of the system by an automated sequence control of pumps 22, 27, 40, 70 and of clamps 30, 52 and 64), the system of FIGURE 1 primes the fluid circuit with saline solution from supply 18.

During the priming sequence, collection needle 12 and return needle 14 will remain housed within antiseptic enclosure 16. Enclosure 16 preferably includes a nonwettable microscopic air filter 72 which, when disposed vertically, permits any included air to exit from the system but which does not permit the entry of microbes nor the exit of saline solution from enclosure 16.

The saline solution from supply 66 may be pumped (in either direction) by the collection and/or return pumps 27, 70, respectively, around a fluid circuit in which the saline solution passes, among others, through the tubing 54 and return needle 14, the common enclosure 16, collection needle 12 and tubing 26.

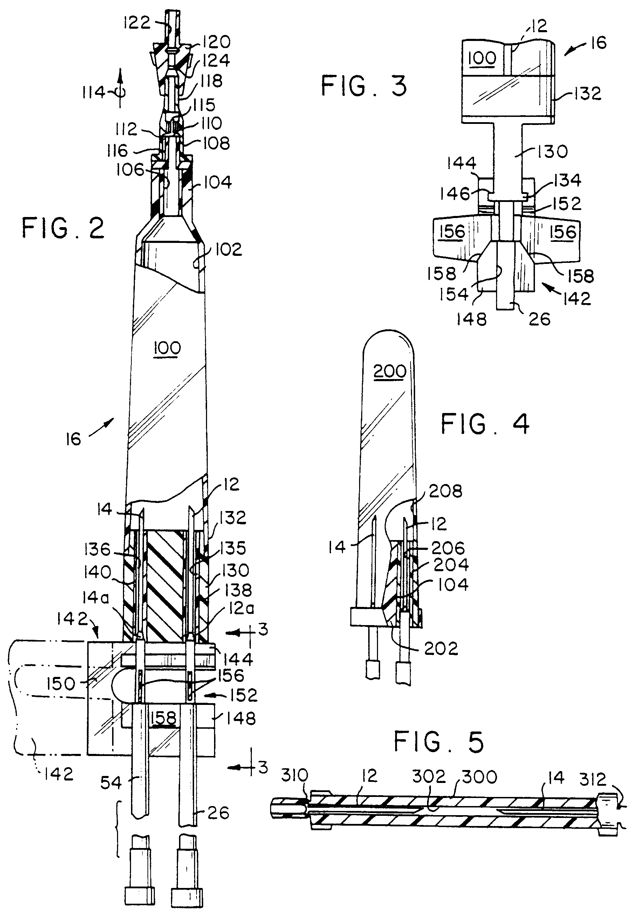

One preferred form of the common needle enclosure 16 is shown in accompanying FIGURES 2 and 3. Here, the needle enclosure 16 generally includes an elongate collection tube 100 defining an interior chamber 102. The distal end 104 of tube 100 also establishes a vent passageway 106 in fluid communication with interior chamber 102. A one-way valve member 108 (of a conventional variety) is axially fixed to distal end 104 and communicates with interior chamber 102. Valve 108 includes a plug member 110 which is displaceable upwardly from valve seat 112 when fluid flows from interior chamber 102 in the direction of arrow 114. Upon such an occurrence, the plug member 110 will be seated against protrusions 115 such that fluid communication is then established between upstream stem 116 and downstream stem 118. On the other hand, when fluid attempts to flow in the opposite direction, plug member 110 will once again be seated against valve seat 112 so as to prevent any fluid flow. Accordingly, one-way valve 108 only permits fluid (e.g., air and/or saline solution) to flow in the direction of arrow 114.

The upstream stem 118 preferably has a male luer-type taper so as to couple with a female luer-type taper on filter member 120. Filter member 120 defines an axial flow path 122 which communicates with chamber 102 via one-way valve 108 but which is interrupted by means of biological filter medium 124. Biological filter medium 124 may be a well-known conventional material which is pervious to air but impervious to microbes and/or liquid. The hydrophobic nature of filter 124 is advantageous in the FIGURE 2 embodiment because air which enters chamber 102 via the collection and return needles 12, 14 may accumulate in the distal end 104 and pass (via one-way valve 108) into passageway 122 and through filter medium 124. Any saline solution, however, which may reach filter medium 124 during the priming sequence is prevented from discharge due to the hydrophobic nature of filter medium 124. Thus, the enclosure 16 conveniently automatically vents only air from the tubing network. At the same time, because at least two needles are involved in this embodiment one may continue to have fluid flow within the network so as to move otherwise trapped air pockets to a discharge point (or to a proper air trap point).

A plug member 130 sealingly closes the proximal end 132 of enclosure 100 and, moreover, includes, at a lower end, a transverse flange 134 (as is seen more clearly by reference to FIGURE 3). Plug member 130 also defines a pair of passageways 135, 136 permitting needles 12, 14 to access interior chamber 102. Of course, if needles additional to needles 12, 14 are required, plug 130 can be suitably modified to include further passageways to accommodate same.

Passageways 135, 136 may each include a liner 138, 140, respectively, of resilient material so as to provide sufficient compliance to sealingly engage proximal shoulders 12a, 14a of needles 12, 14. It should be noted tha needles 12, 14 are coaxially positioned within passageways 135, 136 so as to be spaced from liners 138, 140. This is particularly advantageous since biological lubricants (e.g., silicone) typically coated upon needles 12, 14 will not be removed when needles 12, 14 are withdrawn from passageways 135, 136, respectively.

A generally C-shaped coupling member 142 is provided to retain needles 12, 14 within enclosure 16 so as to prevent inadvertent separation. Coupling member 142 includes an upper leg 144 which defines a slot 146 for slidably receiving transverse flange 134. The slidable cooperation between slot 146 and flange 134 permits coupling member 142 to be moved from a coupled position with plug 130 (and thus enclosure 16 as shown in solid line in FIGURE 2) to an uncoupled position (shown in chain line in FIGURE 2). Coupling number 142 also includes a lower leg 148 integrally connected to upper leg 144 by means of lateral bridge portion 150 so as to define, together with upper leg 144, an open-ended transverse space 152. Lower leg 148 is itself split axially so as to define a transverse trough 154 (see FIGURE 3).

Prior to use, it is important that the collection and return needles 12, 14, respectively, be maintained in the sterile environment of interior chamber 102 and thus, the prevention of inadvertent needle withdrawal is desirable. Coupling member 142 is one example of a structure to achieve such a function. The collection and return needles 12, 14, in and of themselves, may be conventional and may include a pair of conventional grippable radial wings 156 to assist the phlebotomist in the manual manipulation thereof. Coupling member 142 accepts wings 156 within space 152 such that the lower edges of wings 156 bear against the lateral beveled edges 158 of lower leg 148. The tubing 26, 54 connected to the collection and return needles, respectively, is thus positioned within the trough 154 of the lower leg 148. In such a manner, the wings 156 preclude inadvertent withdrawal of the collection and return needles due to their bearing engagement with coupling member 142 when the latter is connected to flange 134. At the proper time, the phlebotomist can slidably remove coupling member 142 from flange 134 (as shown in chain line in FIGURE 2) thereby permitting withdrawal of the collection and/or return needles 12, 14, respectively. As such, only a minimal time period is required in which the collection and return needles 12, 14 are exposed to a nonsterile environment.

FIGURES 4 and 5 each show further examples of the invention. The structure of FIGURE 4 is similar to the structures shown and described above with respect to FIGURES 2 and 3 with the principal exception being that no vent passageway is provided in the distal end of tube 200. A plug member 202 sealably closes the proximal end of tube 200 and, like the embodiment of FIGURES 2 and 3, includes flexible liners 204 for passageways 206 so that needles 12, 14 are each in communication with chamber 208 of enclosure 200.

In FIGURE 5, a common enclosure tube 300 is provided so that the collection and return needles 12, 14 are opposed to one another coaxially yet are each in fluid communication with interior chamber 302. In the embodiment of FIGURE 5, final purging is preferably accomplished in the direction of expected later blood flow to better ensure that entrained air from the tubing network will not later enter the return line. Each of the needles 12, 14 is preferably integrally molded with enclosure tube 300 so as to provide unitary frangible coupling members 310, 312, respectively. Relative twisting between the needles 12, 14 and enclosure tube 300 will thus break the frangible couplings 310, 312 so that the needles 12 and 14 can be withdrawn from tube 300 and then intravenously connected to the patient. (The frangible couplings 310, 312 could also be provided with the embodiment of FIGURES 2-3 and FIGURE 4, if desirable.) Enclosure tube 300 may be rigid or may be flexible, or may include a flexible central region for ease in packaging and tubing routing in use.

Each embodiment of this invention is particularly well suited for automated purging of air from a tubing network since a closed flowpath is provided through the needle(s) later to be used for intravenous connection.

The purging sequence may also include a temporary partial evacuation of the interior chamber defined by the needle enclosure by, for example, operating pump 27 in the proper direction with clamp 52 closed (see FIGURE 1). This may be desirable, for example, to ensure a sufficient liquid column in the pressure transducer branch lines 34, 56. Thus, after drawing a slight vacuum (e.g., 50 mmHg), clamp 52 is released and saline purge solution from supply 66 will positively flow into the tubing system (including a portion of the pressure transducer branch lines). This type of vacuum priming procedure may also assist in filling enclosure 16 via needle 14 and thence on to line 26 via needle 12 to more efficaciously purge air from the tubing network. Air which is forcibly transferred into line 26, may then be conveniently trapped at a suitable location elsewhere in the tubing network, for example, by trap 28 and/or at the top of saline supply 66.

## Claims

1. A method for maintaining the sterility until actual usage of at least one tubular blood collection/return needle (12,14) mounted at the terminal end of a tubing network, said method comprising the step of manufacturing the tubing network as a sterile unit including the needle with the needle removably coupled with a housing (16,200,300) with the needle (12,14) accessing a sterile chamber (102,208,302) of the housing (16,200,300) such that the needle (12,14) is in liquid communication with the chamber (102,208,302); inserting the tubing network unit into a blood processing apparatus, with the needle coupled in liquid communication with the sterile chamber, pumping a sterile liquid into said chamber (102,208,302) through said needle (12,14) and then allowing at least some of said liquid to exit said chamber (102,208,302) and flow back into said network whereby the sterility of the needle (12,14) is maintained until actual usage and the system is purged of air by the liquid flow, and uncoupling the needle from the housing for use.

2. A method according to claim 1 wherein the liquid flow through the chamber (102, 208) 302) is cyclically repeated to ensure that the tubing network is filled completely with said liquid.

3. A method as in any preceding claim wherein air is collected in a distal portion (104) of said chamber (102, 208, 302) during said liquid flow.

4. A method as in claim 3 further comprising the step of venting said collected air from said chamber (102, 208, 302).

5. A method as in any preceding claim wherein the tubing network has at least two tubular needles (12, 14) at the terminal ends thereof; and the liquid flows into the chamber (102, 208, 302) through one needle (14) and exits through a different needle (12) back into the tubing network.

6. A method as in claim 5 wherein the tubing set includes a pressure transducing branch (34, 56) purposefully including a trapped air segment and wherein there is (i) an initial obstruction of liquid flow via one needle (14); (ii) followed by the establishment of a reduced pressure condition within said chamber (102, 208, 302) within a portion of said tubing system including said pressure transducing branch (34, 56) via another needle (12); and then (iii) the removal of the liquid flow obstruction of step (i) to thereby allow liquid to flow into said chamber (102, 208, 302) via said one needle (14) and then on to said other portion of said tubing system via said other needle (12) under influence of said reduced pressure condition established by step (ii) thereby ensuring at least a predetermined minimum column of liquid within said pressure transducing branch (34, 56).

7. Apparatus for use in the method of claim 1, comprising a blood processing system and apparatus, usable for purging air from the blood processing system (10), the apparatus comprising a tubing network which is manufactured as a sterile unit including a collection needle (12) at one terminal end thereof, a return needle (14) at another terminal end thereof, a housing (16,200,300) having a sterile interior chamber (102,208,302), and coupling means removably coupling the needles (12,14) to the housing (16,200,300) so that both needles (12,14) access the same sterile chamber (102,208,302) of the housing (16,200,300) such that flow communication between said chamber (102,208,302) and the needles (12,14) is established, the tubing network unit being connectable to the blood processing system, with the needles coupled in liquid communication with the sterile chamber, whereby passage of a sterile liquid through the tubing network unit including the chamber (102,208,302) to purge air from the network maintains the sterility of the needles (12,14) until actual usage.

8. Apparatus as in claim 7 wherein said coupling means includes frangible connecting means (310, 312) which, upon breakage, permit removal of said needle (12, 14) from said housing (16, 200, 300).

9. Apparatus as in claim 7 or 8 wherein the housing (16, 200, 300) has vent means having a vent passageway (106) in fluid communication with said chamber (102, 208, 302) at said distal end (104) of said housing (16, 200, 300) for venting air from said housing (16, 200, 300) which accumulates in said chamber (102, 208, 302).

10. Apparatus as in claim 7, 8 or 9 wherein said housing (16, 200, 300) includes a biological hydrophobic filter (72, 124) venting air from the housing (16, 200, 300) while preventing the discharge of fluid therefrom and while also preventing the ingress of microbes to said housing (16, 200, 300).

11. Apparatus as in claims 9 or 10 further comprising a one-way valve (108) in said vent passageway (106) which only permits fluid to flow in a venting direction from said interior chamber (102, 208, 302).

12. Apparatus as in claim 11 wherein said one-way valve (108) is positioned upstream of said vent means relative to said venting direction (114) of fluid flow through said vent passageway (106).

13. Apparatus as in any one of claims 7 to 12 where the or each needle (12, 14) includes a radially extending pair of grip members (156), and wherein said coupling means includes:
a closure plug (130, 202) fixed to and closing the proximal end (132) of said housing (16, 200, 300) and including a transverse flange (134) at a lower end thereof;
said closure plug (130) having passageway

## Patentansprüche

1. Verfahren zum Aufrechterhalten der Sterilität bis zum tatsächlichen Gebrauch von mindestens einer rohrförmigen Blutsammel/Rückführungsnadel (12, 14), die an dem Anschlußende eines Schlauchnetzwerkes angebracht ist, wobei das Verfahren folgende Schritte aufweist: Herstellen des Schlauchnetzwerkes als sterile Einheit, einschließlich der Nadel, wobei die Nadel lösbar mit einem Gehäuse (16, 200, 300) verbunden ist, wobei die Nadel (12, 14) Zugang zu einer sterilen Kammer (102, 208, 302) des Gehäuses (16, 200, 300) besitzt, so daß die Nadel (12, 14) in Flüssigkeitsverbindung mit der Kammer (102, 208, 302) ist; Einsetzen des Schlauchnetzwerkes in eine Blutverarbeitungsvorrichtung,
wobei die Nadel in Flüssigkeitsverbindung mit der sterilen Kammer ist; Pumpen einer sterilen Flüssigkeit in die Kammer (102, 208, 302) durch die Nadel (12, 14) und anschließendes Zulassen, daß zumindest ein Teil der Flüssigkeit aus der Kammer (102, 208, 302) austritt und in das Netzwerk zurückfließt, so daß dadurch die Sterilität der Nadel (12, 14) bis zum tatsächlichen Gebrauch aufrechterhalten und durch den Flüssigkeitsdurchfluß Luft aus dem System gespült wird; und Lösen der Nadel von dem Gehäuse zum Gebrauch.

2. Verfahren nach Anspruch 1,
wobei der Flüssigkeitsdurchfluß durch die Kammer (102, 208, 302) zyklisch wiederholt wird, um sicherzustellen, daß das Schlauchnetzwerk vollständig mit der Flüssigkeit gefüllt wird.

3. Verfahren nach einem der vorhergehenden Ansprüche,
wobei währen des Flüssigkeitsdurchflusses Luft in einem distalen Bereich (104) der Kammer (102, 208, 302) gesammelt wird.

4. Verfahren nach Anspruch 3,
das ferner den Schritt des Ablassens der gesammelten Luft aus der Kammer (102, 208, 302) aufweist.

5. Verfahren nach einem der vorhergehenden Ansprüche,
wobei das Schlauchnetzwerk wenigstens zwei rohrförmige Nadeln (12, 14) an seinen Anschlußenden aufweist; und wobei die Flüssigkeit durch die eine Nadel (14) in die Kammer (102, 208, 302) strömt und durch eine andere Nadel (12) zurück in das Schlauchnetzwerk austritt.

6. Verfahren nach Anspruch 5,
wobei der Schlauchsatz einen Druckwandlerzweig (34, 56) aufweist, der absichtlich ein Lufteinschlußsegment enthält; und wobei (i) eine anfängliche Hemmung von Flüssigkeitsdurchfluß durch die eine Nadel (14) vorhanden ist; (ii) gefolgt von der Ausbildung eines Zustandes verminderten Druckes in der Kammer (102, 208, 302) innerhalb eines Bereiches des Schlauchsystems, der den Druckwandlerzweig (34, 56) enthält, durch eine andere Nadel (12); und anschließend (iii) ein Beseitigen der Flüssigkeitsdurchflußhemmung von Schritt (i) erfolgt, um es dadurch zu ermöglichen, daß Flüssigkeit durch die eine Nadel (14) in die Kammer (102, 208, 302) strömt und dann weiter durch die andere Nadel (12) zu dem anderen Bereich des Schlauchsystems strömt, und zwar unter dem Einfluß des Zustandes verminderten Druckes, der beim Schritt (ii) ausgebildet worden ist, so daß zumindest eine vorbestimmte minimale Flüssigkeitssäule in dem Druckwandlerzweig (34, 56) gewährleistet ist.

7. Vorrichtung zur Verwendung bei dem Verfahren nach Anspruch 1, die ein Blutverarbeitungssystem und eine Vorrichtung aufweist, verwendbar, um Luft aus dem Blutverarbeitungssystem (10) zu spülen, wobei die Vorrichtung folgendes aufweist: ein Schlauchnetzwerk, das als sterile Einheit hergestellt ist, einschließlich einer Sammelnadel (12) an dem einen Anschlußende davon und einer Rückführnadel (14) an ihrem anderen Anschlußende davon; ein Gehäuse (16, 200, 300) mit einer sterilen Innenkammer (102, 208, 302); und eine Verbindungseinrichtung, die die Nadeln (12, 14) lösbar mit dem Gehäuse (16, 200, 300) verbindet, so daß beide Nadeln (12, 14) Zugang zu derselben sterilen Kammer (102, 208, 302) des Gehäuses (16, 200, 300) haben, so daß eine Strömungsverbindung zwischen der Kammer (102, 208, 302) und den Nadeln (12, 14) ausgebildet wird, wobei die Schlauchnetzwerkeinheit an das Blutverarbeitungssystem anschließbar ist, wobei die Nadeln (12, 14) in Flüssigkeitsverbindung mit der sterilen Kammer angeschlossen sind, so daß der Durchfluß einer sterilen Flüssigkeit durch die Schlauchnetzwerkeinheit, einschließlich der Kammer (102, 208, 302), um Luft aus dem Netzwerk zu spülen, die Sterilität der Nadeln (12, 14) bis zum tatsächlichen Gebrauch aufrechterhält.

8. Vorrichtung nach Anspruch 7,
wobei die Verbindungseinrichtung abbrechbare Verbindungsmittel (310, 312) aufweist, die beim Abbrechen das Abnehmen der Nadel (12, 14) von dem Gehäuse (16, 200, 300) ermöglichen.

9. Vorrichtung nach Anspruch 7 oder 8,
wobei das Gehäuse (16, 200, 300) eine Entlüftungseinrichtung mit einem Entlüftungskanal (106) in Fluidverbindung mit der Kammer (102, 208, 302) an dem distalen Ende (104) des Gehäuses (16, 200, 300) aufweist, um Luft aus dem Gehäuse (16, 200, 300) abzulassen, die sich in der Kammer (102, 208, 302) sammelt.

10. Vorrichtung nach Anspruch 7, 8 oder 9,
wobei das Gehäuse (16, 200, 300) einen biologischen hydrophoben Filter (72, 124) aufweist, der Luft aus dem Gehäuse (16, 200, 300) ableitet, während er zugleich den Abfluß von Fluid aus diesem verhindert und während er zugleich außerdem das Eindringen von Mikroben in das Gehäuse (16, 200, 300) verhindert.

11. Vorrichtung nach Anspruch 9 oder 10,
die ferner ein Einwegventil (108) in dem Entlüftungskanal (106) aufweist, das es nur zuläßt, daß Fluid in eine Entlüftungsrichtung aus der Innenkammer (102, 208, 302) strömt.

12. Vorrichtung nach Anspruch 11,
wobei das Einwegventil (108) stromaufwärts von der Entlüftungseinrichtung relativ zu der Entlüftungsrichtung (114) des Fluiddurchflusses durch den Entlüftungskanal (106) positioniert ist.

13. Vorrichtung nach einem der Ansprüche 7 bis 12,
wobei die oder jede Nadel (12, 14) ein radial verlaufendes Paar von Greifelementen (156) aufweist und wobei die Verbindungseinrichtung folgendes aufweist:
einen Verschlußstopfen (130, 202), der an dem proximalen Ende (132) des Gehäuses (16, 200, 300) befestigt ist und es verschließt und einen Querflansch (134) an seinem unteren Ende aufweist;
wobei der Verschlußstopfen (130) einen Durchgang (135, 136, 206) pro Nadel (12, 14) hat, der in Axialrichtung relativ zu dem Gehäuse (16, 200, 300) verläuft, um jeweils eine gesonderte Nadel (12, 14) so zu halten, daß jede Nadel (12, 14) in gemeinsamer Fluidverbindung mit der Innenkammer (102, 208, 302) ist;
wobei jeder der Durchgänge (135, 136, 206) nachgiebige Dichteinrichtungen (138, 140, 204) aufweist, um eine Fluiddichtung mit einem externen Umfangsbereich seiner jeweils zugeordneten Nadel herzustellen und dadurch die Innenkammer (102, 208, 302) gegen das Eindringen von kontaminierenden Substanzen aus der Umgebung abzudichten;
wobei ein im allgemeinen C-förmiges Halteelement (142) ein Paar von getrennten Armen (144, 148) hat, die dazwischen einen Raum (152) bilden, wobei ein oberer Arm dieser Arme eine Führungsausnehmung (146) aufweist, die den Flansch (134) verschiebbar aufnimmt, um es zu ermöglichen, daß das Halteelement (142) mit dem Verschlußstopfen (130) verschiebbar verbunden und von diesem entfernt werden kann;
wobei das Paar von Greifelementen (156) in dem Raum (152) angeordnet ist, der zwischen dem Paar von Armen (144, 148) gebildet ist, so daß die Greifelemente an einem unteren Arm (148) der Arme anliegen, wenn das Halteelement (142) mit dem Gehäuse (16, 200, 300) verbunden ist, um ein versehentliches Herausziehen von der oder jeder Nadel (12, 14) aus ihrem jeweiligen Durchgang (135, 136, 206) zu verhindern.

## Revendications

1. Procédé de conservation de la stérilité jusqu'à l'utilisation effective d'au moins une aiguille tubulaire (12, 14) de prélèvement/retour de sang montée à l'extrémité terminale d'un réseau de tubes, ledit procédé comprenant l'opération de fabrication du réseau de tubes en tant qu'unité stérile comprenant l'aiguille, l'aiguille étant couplée de manière séparable avec une gaine (16, 200, 300) de sorte que l'aiguille (12, 14) accède à une chambre stérile (102, 208, 302) de la gaine (16, 200, 300) d'une manière telle que l'aiguille (12, 14) est en communication de liquide avec la chambre (102, 208, 302) ; d'insertion de l'unité de réseau de tubes dans un appareil de traitement du sang de sorte que l'aiguille soit couplée en communication de liquide avec la chambre stérile, de pompage d'un liquide stérile dans ladite chambre (102, 208, 302) par l'intermédiaire de ladite aiguille (12, 14) puis d'écoulement d'au moins une certaine quantité dudit liquide à la sortie de ladite chambre (102, 208, 302) et d'écoulement en retour dans ledit réseau de sorte que la stérilité de l'aiguille (12, 14) est maintenue jusqu'à l'utilisation effective et que le système est purgé de l'air qu'il contenait, par l'écoulement de liquide, et de découplage de l'aiguille hors de sa gaine pour l'utilisation.

2. Procédé suivant la revendication 1, dans lequel l'écoulement de liquide à travers la chambre (102, 208, 302) est cycliquement répété pour assurer le remplissage complet du réseau de tubes par ledit liquide.

3. Procédé suivant l'une quelconque des revendications précédentes, dans lequel l'air est collecté dans une partie distale (104) de ladite chambre (102, 208, 302) pendant ledit écoulement de liquide.

4. Procédé suivant la revendication 3, comprenant en outre l'opération d'évacuation à l'atmosphère dudit air collecté, à partir de ladite chambre (102, 208, 302).

5. Procédé suivant l'une quelconque des revendications précédentes, dans lequel le réseau de tubes comporte au moins deux aiguilles tubulaires (12, 14) à ses extrémités terminales, et le liquide pénètre dans la chambre (102, 208, 302) par une aiguille (14) et sort par une aiguille différente (12) pour revenir dans le réseau de tubes.

6. Procédé suivant la revendication 5, dans lequel le réseau de tubes comprend un branchement de transduction de pression (34, 56) comportant volontairement un segment de piégeage d'air, et dans lequel il y a (i) une obstruction initiale de l'écoulement de liquide par l'intermédiaire d'une première aiguille (14) ; (ii) suivie par l'établissement d'une pression réduite dans ladite chambre (102, 208, 302), dans une partie dudit réseau de tubes comportant ledit branchement de transduction de pression (34, 56), par l'intermédiaire d'une autre aiguille (12) ; puis (iii) la suppression de l'obstruction d'écoulement de liquide de l'étape (i) pour permettre ainsi au liquide de pénétrer dans ladite chambre (102, 208, 302) par l'intermédiaire de ladite première aiguille (14) puis de continuer à s'écouler vers ladite autre partie dudit réseau de tubes par l'intermédiaire de ladite autre aiguille (12) sous l'influence de ladite pression réduite établie par l'état (ii), de façon à assurer au moins une colonne minimale prédéterminée de liquide dans ledit branchement de transduction de pression (34, 56).

7. Appareil utilisable dans le procédé de la revendication 1, comprenant un système et un appareil de traitement de sang utilisable pour purger l'air du système de traitement du sang (10), l'appareil comprenant un réseau de tubes fabriqué en tant qu'unité stérile comportant une aiguille de prélèvement (12) à une de ses extrémités et une aiguille de retour (14) à une autre de ses extrémités ; une gaine (16, 200, 300) comportant une chambre intérieure stérile (102, 208, 302) ; et des moyens de couplage pour relier de façon séparable les aiguilles (12, 14) à la gaine (16, 200, 300) de sorte que les deux aiguilles (12, 14) accèdent à la chambre stérile (102, 208, 302) de la gaine (16, 200, 300) afin d'établir une communication de fluide entre ladite chambre (102, 208, 302) et les aiguilles (12, 14), l'unité de réseau de tubes étant connectable au système de traitement du sang, de sorte que les aiguilles soient couplées en communication de liquide avec la chambre stérile, moyennant quoi le passage d'un liquide stérile dans le réseau de tubes et la chambre (102, 208, 302) pour purger l'air du réseau maintient la stérilité des aiguilles (12, 14) jusqu'à l'utilisation effective.

8. Appareil suivant la revendication 7, dans lequel lesdits moyens de couplage comprennent des moyens de connexion cassables (310, 312) qui permettent, après cassure, de retirer ladite aiguille (12, 14) de ladite gaine (16, 200, 300).

9. Appareil suivant la revendication 7 ou 8, dans lequel la gaine (16, 200, 300) comporte des moyens de mise à l'atmosphère ayant un passage d'évent (106) en communication de fluide avec ladite chambre (102, 208, 302), situé à ladite extrémité distale (104) de ladite gaine (16, 200, 300) pour purger à l'atmosphère l'air de ladite gaine (16, 200, 300) qui s'accumule dans ladite chambre (102, 208, 302).

10. Appareil suivant la revendication 7, 8 ou 9, dans lequel ladite gaine (16, 200, 300) comprend un filtre biologique hydrophobe (72, 124) qui purge à l'atmosphère l'air de la gaine (16, 200, 300) tout en empêchant la sortie de fluide de la gaine et en empêchant également l'entrée de microbes dans ladite gaine (16, 200, 300).

11. Appareil suivant la revendication 9 ou 10, comprenant en outre un clapet unidirectionnel (108), placé dans ledit passage d'évent (106), qui permet seulement l'écoulement de fluide dans une direction de purge de ladite chambre intérieure (102, 208, 302).

12. Appareil suivant la revendication 11, dans lequel ledit clapet unidirectionnel (108) est placé en amont desdits moyens de mise à l'atmosphère, avec référence à ladite direction de purge (114) de l'écoulement de fluide dans ledit passage d'évent (106).

13. Appareil suivant l'une quelconque des revendications 7 à 12, dans lequel la ou chaque aiguille (12, 14) comporte deux éléments de préhension (156) s'étendant radialement, et dans lequel lesdits moyens de couplage comprennent :
un bouchon (130, 202) fermant l'extrémité proche (132) de ladite gaine (16, 200, 300), et comportant une collerette transversale (134) à son extrémité inférieure ;
ledit bouchon (130) comportant un passage (135, 136, 206) par aiguille (12, 14), s'étendant axialement par rapport à ladite gaine (16, 200, 300), chaque passage pouvant retenir une aiguille séparés (12, 14) de sorte que chaque aiguille (12, 14) est en communication commune de fluide avec ladite chambre intérieure (102, 208, 302) ;
chaque dit passage (135, 136, 206) comportant des moyens d'étanchéité souples (138, 140, 204) pour établir une étanchéité au fluide avec une région périphérique externe de son aiguille respectivement associée, de manière à isoler ladite chambre intérieure (102, 208, 302) contre la pénétration de contaminants ambiants ;
une pièce de retenue sensiblement en forme de C (142) comportant deux branches séparées (144, 148) qui définissent entre elles un espace (152), une branche supérieure desdites branches comportant un logement de guidage (146) qui reçoit de façon coulissante ladite collerette (134) pour permettre à ladite pièce de retenue (142) d'être reliée de façon coulissante audit bouchon (130) et retirée de celui-ci ;
lesdits deux éléments de préhension (156) étant disposés dans ledit espace (152) défini entre lesdites deux branches (144, 148) de sorte que lesdits éléments de préhension s'appuient contre une branche inférieure des dites branches (148) lorsque ladite pièce de retenue (142) est accouplée à ladite gaine (16, 200, 300), pour empêcher l'extraction accidentelle de l'aiguille ou de chaque aiguille (12, 14) de son passage respectif (135, 136, 206).
